# EUROPEAN PATENT APPLICATION

(11) **EP 1 832 572 A1**
(43) Date of publication of application: **12.09.2007**
(21) Application number: 06004756.0
(22) Date of filing: 08.03.2006
(51) Int. Cl.: C07C 213/10, C07C 215/40

(54) **Process for the enantiomeric enrichment of salbutamol and salbutamol precursors**

(71) Applicant: Stirling Products Limited, Perth WA 6000 (AU)
(72) Inventor: Reuter, Karl, 79194 Gundelfingen (DE); Meier, Viktor, 79194 Gundelfingen (DE); Stolz, Florian, 79104 Freiburg (DE)
(74) Representative: Reitstötter - Kinzebach

(57) **Abstract**

The present invention relates to a process for the enantiomeric enrichment of salbutamol and salbutamol precursors and the acid-addition salts thereof: 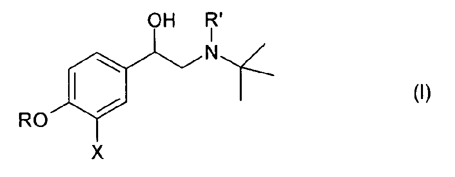 where R is hydrogen or benzyl, R' is hydrogen or benzyl and X is CH₂OH or COO-C₁-C₄-alkyl
the process comprising the crystallization of a compound of the formula I in the form of its acid-addition salt with an achiral carboxylic acid A that has at least three carbon atoms and a solubility of less than 50 g/l in water at pH < 3, 20°C and 1013 mbar, from a solution containing a mixture of the enantiomers of the compound of formula I and the achiral carboxylic acid A in the presence of seed crystals of the desired enantiomer of the compound of formula I or the acid addition salt thereof, whereby the enantiomerically enriched acid-addition salt of the compound of formula I is obtained.

## Description

### BACKGROUND OF THE INVENTION

The present invention relates to a process for the enantiomeric enrichment of salbutamol and salbutamol precursors and the acid-addition salts thereof. The invention also relates to specific acid-addition salts of salbutamol and salbutamol precursors that are useful for obtaining single enantiomers of these compounds.

Salbutamol (also referred to as albuterol or α-[[(1,1-dimethylethyl)amino]methyl]-4-hydroxy-1,3-benzenedimethanol) is a β₂-agonist useful as a bronchodilator and is thus used in the treatment of respiratory diseases such as asthma and related medical conditions. It is known that the (R) isomer of salbutamol (which is laevorotatory) is about 80 times more active than the dextrorotatory (S) isomer, and research has indicated that administration of the pure (R) enantiomer may offer an improved therapeutic ratio. Recently, the use of the (R) enantiomer of salbutamol as a feed additive has been suggested (US6110974). Therefore, much effort has been spent to provide the pure (R) enantiomer of salbutamol.

EP 763010 discloses a process for obtaining single enantiomers of salbutamol including the optical resolution of methyl 5-[2-[(1,1-dimethylethyl)amino]-1-hydroxyethyl]-2-(phenylmethoxy)benzoat or α-[[(1,1-dimethylethyl)amino]methyl]-4-(phenylmethoxy)-1,3-benzenedimethanol by means of a chiral tartaric acid derivative such as (-)-di-toluoyl-L-tartaric acid and (+)-di-toluyl-D-tartaric acid in methanol.

US 6,365,756 describes the resolution of a new ketal derivative of salbutamol (specifically 2-(N-t-butylamino)-1-(2,2-dimethyl-1,2-benzodioxin-6-yl)ethanol). The resolution is again performed using a chiral tartaric acid derivative.

Chiral tartaric acid derivatives, however, are expensive and, therefore, the use of these compounds renders these processes unattractive from an economical point of view. Moreover, the reported enantiomeric excess is not satisfactory.

EP 1349828 describes a process for obtaining pure (R) salbutamol or pharmaceutically acceptable salts thereof by resolving a racemic mixture of salbutamol or salbutamol precursors with (L) tartaric acid and, where necessary, converting the precursor into salbutamol. Again, however, an expensive chiral auxiliary has to be used and the overall yield for optical resolution of salbutamol is not satisfactory.

Argentine Patent Application No. 990102613 discloses a process for the optical resolution of a racemic mixture of salbutamol sulfate. The process includes the enrichment of racemic salbutamol sulfate with the desired enantiomer of salbutamol sulfate, forming a supersaturated solution of the thus enriched salbutamol sulfate and inducing crystallization by seeding with small amounts of the desired enantiomer of salbutamol sulfate. The process is not satisfactory for several reasons. First, large amounts of solvent are required, whereby the space-time-yield of the crystallization is lowered. Second, the reported enantiomeric enrichment is not satisfactory despite the prior enrichment with the desired enantiomer.

Therefore, there is still a strong need for providing a process for enantiomeric (optical) enrichment of salbutamol and salbutamol precursors that overcomes the disadvantages of the prior art.

### SUMMARY OF THE INVENTION

Surprisingly, it has been found that this object is solved by "preferential crystallization" of acid-addition salts of salbutamol or salbutamol precursors of the formula I as defined herein with an achiral carboxylic acid A that has at least three carbon atoms and a solubility of less than 50 g/L in water at pH < 3, 20° C and 1013 mbar.

Salbutamol and salbutamol precursors can be described by the formula I 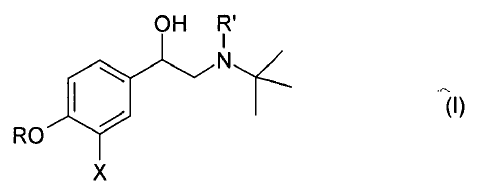 where R is hydrogen or benzyl, R' is hydrogen or benzyl and X is CH₂OH or COO-C₁-C₄-alkyl.

The preferential crystallization includes the crystallization of a compound of the formula I in the form of its acid-addition salt with the achiral carboxylic acid A from a solution containing a mixture of the enantiomers of the compound of formula I and the achiral carboxylic acid A in the presence of seed crystals of the desired enantiomer of the respective compound of the formula I or the acid addition salt thereof.

Therefore, the present invention relates to a process for the enantiomeric enrichment of a compound of the formula I or an acid-addition salt thereof by the process of preferential crystallization as described herein.

The enantiomeric enrichment of salbutamol (compound I, wherein R and R' are hydrogen and X is CH₂OH) is a preferred embodiment of the present invention.

The enantiomeric enrichment of 4- benzyl salbutamol (compound I, wherein R is benzyl, R' is hydrogen and X is CH₂OH) is a further preferred embodiment of the present invention.

Further embodiments of the invention relate to the enantiomeric enrichment of methyl 5-[2-[(1,1-dimethylethyl)amino]-1-hydroxyethyl]-2-hydroxybenzoat (compound I, wherein R and R' are hydrogen and X is CO₂CH₃), methyl 5-[2-[(1,1-dimethylethyl)amino]-1-hydroxyethyl]-2-benzyloxybenzoat (compound I, wherein R is benzyl R' is hydrogen and X is CO₂CH₃) and methyl 5-[2-[N-benzyl-N-(1,1-dimethylethyl)amino]-1-hydroxyethyl]-2-benzyloxybenzoat (compound I, wherein R and R' are benzyl and X is CO₂CH₃).

The invention also relates to the novel acid-addition salts of the compound of formula I with said carboxylic acid A, in particular to enantiomerically enriched or pure acid-addition salts of the compound of formula I with the carboxylic acid A.

The use of the acid-addition salt of a compound of formula I with the carboxylic acid A provides good crystallization yields and satisfactory enantiomeric enrichment generally exceeding 70 : 30, in particular 80 : 20, without using expensive chiral auxiliaries. Satisfactory enrichment can be obtained without prior enrichment of racemic salbutamol to be resolved. The process of the present invention also allows the use of high concentrations of a compound of formula I without adversely affecting the enantiomeric enrichment. Moreover, the crystallization of the acid-addition salt of I can be carried out in an aqueous solution or emulsion, which renders the process more economical and less risky, since expensive or dangerous organic solvents can be reduced or avoided.

### DETAILED DESCRIPTION OF THE INVENTION

According to the present invention, a compound of formula I, in particular salbutamol or 4-benzyl salbutamol, is crystallized as its acid-addition salt with an achiral carboxylic acid A. Suitable carboxylic acids A include mono- and dicarboxylic acids having at least three carbon atoms and a water solubility of less than 50 g/L in water at pH < 3, 20 °C and 1013 mbar. Preferably the carboxylic acid has a water solubility of at least 0.1 g/L in water at pH < 3, 20 °C and 1013 mbar. Preferably, the carboxylic acid A is selected from monocarboxylic acids, in particular from alkanoic acids having from 4 to 20 carbon atoms, benzoic acids and naphthoic acids, wherein the benzene or naphthene ring may be unsubstituted or may carry 1, 2 or 3 radicals selected independently of each other from halogen, alkyl, cyano, nitro and alkoxy.

The term "alkyl" as used herein includes linear or branched alkyl groups having preferably from 1 to 4, in particular 1 or 2 carbon atoms, such as methyl, ethyl, n-propyl, isopropyl, n-butyl, isobutyl, 2-butyl and 1,1-dimethylethyl (= tert.-butyl).

The term "alkoxy" as used herein includes linear or branched alkoxy radicals having preferably from 1 to 4, in particular 1 or 2 carbon atoms, such as methoxy, ethoxy, n-propoxy, isopropoxy, n-butoxy, 2-butoxy, isobutoxy und 1,1-dimethylethyloxy.

In a preferred embodiment, the carboxylic acid A is an alkanoic acid having from 4 to 20 carbon atoms, preferably from 4 to 10 carbon atoms, in particular an α-branched alkanoic acid such as pivalic acid. Alkanoic acids, in particular α-alkanoic acids, such as pivalic acid, are especially useful for the enantiomeric enrichment of salbutamol.

In a second preferred embodiment, the carboxylic acid is a benzoic acid or naphthoic acid which may be unsubstituted or substituted as mentioned above. In this embodiment, particular preference is given to benzoic acids, which may be unsubstituted or substituted as mentioned above and which in particular carry 1 or 2 radicals selected from chloro, fluoro, methyl, cyano, nitro, hydroxy and methoxy. Particular preference is given to 3,5-dinitrobenzoic acid. The carboxylic acid A of this second preferred embodiment is particularly useful for the enantiomeric enrichment of 4-benzyl salbutamol.

According to the invention, the crystallization of the acid-addition salt of the compound of formula I is carried out in the presence of seed crystals of the desired enantiomer of the respective compound of formula I or of its acid-addition salt, in particular in the presence of seed crystals of its acid addition salt with the carboxylic acid A.

The desired enantiomer is generally the R-enantiomer of the compound of formula I. The invention, however, can also be used to enrich the S-enantiomer, which is then the desired enantiomer. If the R-enantiomer is the desired enantiomer, however, the S-enantiomer is the non-desired enantiomer.

The crystallization of the acid addition salt of desired enantiomer of I is induced by the presence of seed crystals of the desired enantiomer or its acid-addition salt. Usually, these seed crystals are provided by addition of seed crystals of the acid-addition salt of the desired enantiomer to the solution containing a mixture of the enantiomers of formula I and the achiral carboxylic acid A. Preferbly, the mixture of enantiomers is a racemic mixture of the respective compound of formula I. However, it is also possible to use a non-racemic mixture of the enantiomers of respective compound of formula I, or of its acid-addition salt, in which the desired enantiomer has been enriched (or the non-desired enantiomer has been depleted) prior to crystallization. To this non-racemic mixture seed crystals of the desired enantiomer or its acid-addition salt can be added or the seed crystals may be generated in situ, if the desired enantiomer in the mixture to be resolved is present in excess. In a preferred embodiment of the invention, crystallization is effected from a solution of the racemic mixture of the compound of formula I or of its acid-addition salt by addition of seed crystals of the desired enantiomer.

If a non-racemic mixture of the enantiomers of formula I is used for crystallization, the excess of the desired enantiomer usually does not exceed 30 %, in particular 20 %, i. e. the molar ratio of desired enantiomer to non-desired enantiomer does not exceed 65 : 35, in particular 60 : 40. Such non-racemic mixtures may be obtained during crystallization of the desired enantiomer of the acid-addition salt from a solution of the racemic mixture of the compound of formula I according to the process of the present invention. During this process, the desired enantiomer is depleted and the non-racemic mixture of the enantiomers of formula I is contained in the mother liquor. The mother liquor or the non-racemic mixture of enantiomers isolated therefrom may be used in a subsequent crystallization of the acid-addition salt of the other enantiomer. It is also possible to add seed crystals of the desired enantiomer to a solution of the non-racemic mixture containing an excess of the desired enantiomer. Preferably, seed crystals are used both for the crystallization from a solution of the racemic mixture of a compound of formula I and for non-racemic mixtures.

According to the invention, the crystallization of the acid-addition salt of the compound of formula I is performed in the presence of the carboxylic acid A. Needless to say, the amount of carboxylic acid A is chosen to ensure crystallization of the acid-addition salt of the compound of formula I. Usually, the amount of achiral carboxylic acid A present is close to the required stoichiometry of the acid-addition salt of compound I and generally does not deviate from the required stoichiometry by more than 20 mol-%. In particular, the molar ratio of carboxylic acid A to the compound of formula I in the solution before crystallization is from 0.9 : 1 to 1.1 : 1, in particular from 0.95 : 1 to 1.05 : 1. The necessary amount of the carboxylic acid A can be added to a solution of the compound of formula I or vice versa. Thereby, a solution of the acid-addition salt is formed. Alternatively, a solution containing the compound I and the carboxylic acid A can by provided by dissolving the acid-addition salt of the compound of formula I.

The concentration of the compound of the formula I in the solution before crystallization is usually from 25 to 700 g/L, preferably from 50 to 650 g/L, in particular from 100 to 500 g/L, or 150 to 450 g/L calculated as the free base of I.

The crystallization of the acid-addition salt of the compound of formula I can be performed by analogy to well-known crystallization techniques. Their basic premise is the formation of a solution containing the mixture of the enantiomers of the compound of formula I and the achiral carboxylic acid A, supersaturation by conventional techniques, and subsequent induction of the crystallization of the desired enantiomer. The crystallization can be induced by adding seed crystals of the desired enantiomer, preferably in the form of its acid-addition salt, by further lowering the solubility of the acid-addition salt and/or by scratching or ultrasound. Lowering the solubility of the acid-addition salt can be achieved by lowering the temperature, adding a non-solvent to the solution and/or by adding further achiral carboxylic acid A or compound of formula I.

Preferably, crystallization is induced by addition of seed crystals of the acid-addition salt of the desired enantiomer. The amount of seed crystals added to the solution of the mixture of enantiomers is preferably from 0.1 to 10 mmol, preferably from 0.2 to 8 mmol, in particular from 0.5 to 5 mmol, per mol of the compound of formula I contained in the solution.

It has been proven advantageous, if the crystallization is performed in the presence of at least one surface active compound, which, below, is also termed as surfactant. Suitable surfactants include non-ionic, anionic, cationic and zwitterionic surfactants and mixtures thereof. In a preferred embodiment, the surfactant will be present in an amount ranging from 0.1 to 30 % by weight, based on the total weight of the solution before crystallization, in particular from 1 to 20 % by weight.

Anionic surfactants include in particular the sodium, potassium calcium or ammonium salts of
- C₆-C₂₂-alkylsulfonates such as lauryl sulfonate, isotridecylsulfonate;
- C₆-C₂₂-alkylsulfates such as lauryl sulfate, isotridecylsulfate, cetylsulfate, stearylsulfate;
- aryl- and C₁-C₁₆-alkylarylsulfonates such as naphthylsulfonate, dibutylnaphtylsulfonate, dodecyldiphenylether sulfonate, cumylsulfonate, nonylbenzenesulfonate, dodecylbenzene sulfonate;
- sulfates and sulfonates of fatty acids and fatty acid esters;
- sulfates of ethoxylated C₆-C₂₂ alkanoles such as sulfates of ethoxylated lauryl alcohol;
- sulfates of ethoxylated C₁-C₁₆-alkylphenols;
- mono- and diesters of phosphorous acid, including mixtures thereof with triesters and salts thereof, in particular the esters with C₈-C₂₂-alkanols, ethoxylated C₈-C₂₂-alkanols, C₄-C₂₂-alkylphenols, ethoxylated C₄-C₂₂-alkylphenols, di- or tristyrylphenols, ethoxylated di- or tristyrylphenols;
- di C₄-C₁₆ alkylesters of sulfosuccinic acid such as dioctylsulfosuccinate;
- acylsarcosinates;
- fatty acids such as stearates;
- acylglutamates; and
- condensates of naphthalinesulfonic acid or phenolsulfonic acid with formaldehyde.

Non-ionic surfactants include in particular
- polyethyleneglycolalkylethers, polyethyleneglycol/polypropyleneglycolalkylethers, in particular polyethoxylates and poly-ethoxylates-co-propoxylates of linear or branched C₈-C₂₀-alkanoles, more preferably polyethoxylated fatty alcohols and polyethoxylated oxoalcohols, such as polyethoxylated lauryl alcohol, polyethoxylated isotridecanol, polyethoxylated cetyl alcohol, polyethoxylated stearyl alcohol, and esters thereof, such as acetates;
- polyethylenglycol arylethers, in particular polyethoxylates of C₁-C₁₆-alkylphenoles, such as polyethoxylates of nonylphenol, decylphenol, isodecylphenol, dodecylphenol or isotridecylphenol, polyethoxylates of mono-, di- und tristyrylphenoles; and the esters thereof, e.g. the acetates;
- C₆-C₂₂-alkylglucosides and C₆-C₂₂-alkyl polyglucosides;
- polyethoxylates of C₆-C₂₂-alkylglucosides and polyethoxylates of C₆-C₂₂-alkyl polyglucosides;
- polyethoxylates of fatty amines;
- polyethoxylates of fatty acids and polyethoxylates of hydroxyl fatty acids;
- partial esters of polyols with C₆-C₂₂-alkanoic acids, in particular mono- and diesters of glycerine and mono-, di- and triesters of sorbitan, such as glycerine monostearate, sorbitanmonooleat, sorbitantristearat;
- polyethoxylates of partial esters of polyols with C₆-C₂₂-alkanoic acids, in particular polyethoxylates of mono- and diesters of glycerine and polyethoxylates of mono-, di- and triesters of sorbitan, such as polyethoxylates of glycerine monostearate, polyethoxylates of sorbitanmonooleat, polyethoxylates of sorbitanmonostearat and polyethoxylates of sorbitantristearat;
- polyethoxylates of vegetable oils or animal fats such as corn oil ethoxylate, castor oil ethoxylate, tallow oil ethoxylate;
- triesters of phosphorous acid, in particular the triesters with C₈-C₂₂-alkanols, ethoxylated C₈-C₂₂-alkanols, C₄-C₂₂-alkylphenols, ethoxylated C₄-C₂₂-alkylphenols, di- or tristyrylphenols, ethoxylated di- or tristyrylphenols;
- polyoxyethylene-polyoxypropylene-blockcopolymers; and
- polyethoxylates of fatty amines, fatty amides or of fatty acid diethanolamides.

Cationic surfactants include in particular
- protonated mono-, di- and trialkylammonium compounds and quaternary tetralkylammonium compounds, in particular C₆-C₂₂-alkyltrimethylammonium salts and di-C₆-C₂₂-alkyldimethylammonium salts, e.g. the halides, sulfates and alkylsulfates, including protonated or quarternized polyethoxylates of mono-, di- and trialkylamines;
- alkyl pyridinium salts, in particular C₆-C₂₂-alkylpyridinium salts e.g. the halides, sulfates and C₁-C₄-alkylsulfates; and
- alkyl and dialkyl imidazolinium salts, in particular N,N'- C₆-C₂₂-dialkylimidazolinium salts, e.g. the halides, sulfates or methoxysulfates.

Zwitterionic (amphoteric) surfactants include in particular
- aminoxides of tertiary alkylamines; and
- betaines, in particular 2-(trialkylamonium)acetic acid or 2-(N-(alkylcarbonylaminoalkyl)-N,N-dialkylamino)acetic acid or coco-beta-aminobutyric acid.

The terms polyethyleneglycol and polyethoxylates refer to polyether radicals derived from ethyleneoxide. Likewise, the term polyoxyethylene-co-polyoxypropylene refers to a polyether radical derived from a mixture of ethyleneoxide and propylenoxide. The number of repeating units in the polyether radicals will generally vary from 4 to 100, in particular from 5 to 50.

Preferably, the surfactant, if present, comprises at least 50 % by weight, based on the total amount of surfactant present, of at least one non-ionic surfactant. In particular, the surfactant comprises at least 80 %, based on the total amount of surfactant, of at least one non-ionic surfactant.

Among the non-ionic surfactants preferred are those which have an HLB-value of at least 10, in particular from 10 to 20. Preferred non-ionic surfactants include
- polyethyleneglycolalkylethers, polyethyleneglycol/polypropyleneglycolalkylethers, in particular polyethoxylates and poly-ethoxylates-co-propoxylates of linear or branched C₈-C₂₀-alkanoles, more preferably polyethoxylated fatty alcohols and polyethoxylated oxoalcohols, such as polyethoxylated lauryl alcohol, polyethoxylated isotridecanol, polyethoxylated cetyl alcohol, polyethoxylated stearyl alcohol;
- polyethylenglycol arylethers, in particular polyethoxylates of C₁-C₁₆-alkylphenoles, such as polyethoxylates of nonylphenol, decylphenol, isodecylphenol, dodecylphenol or isotridecylphenol, polyethoxylates of mono-, di- und tristyrylphenoles;
- polyoxyethylenesorbitanesters of C₈-C₂₂-aliphatic acids, such as polyethoxylates of sorbitanmonooleat, polyethoxylates of sorbitanmonostearat and polyethoxylates of sorbitantristearat;
- polyethoxylates of vegetable oils or animal fats, such as corn oil ethoxylate, castor oil ethoxylate, tallow oil ethoxylate;
- polyethoxylates of mono- or diglycerides of fatty acids, such as polyethoxylates of glycerine monooleate or glycerine monostearate;
- polyoxyethylene-polyoxypropylene-blockcopolymers; and
- polyethoxylates of fatty acids and polyethoxylates of hydroxyl fatty acids such as polyethoxylates of hydroxystearic acid.

Particular preference is given to polyethyleneglycolsorbitan fatty acid esters, polyethyleneglycol C₈-C₂₀-alkylethers, polyethoxylates of C₄-C₁₀-alkylphenoles, ethoxylated castor oil and polyethyleneglycolhydroxystearat.

According to the invention, the crystallization of the acid addition salt is performed from a solution of the mixture of enantiomers of the compound of formula I and the carboxylic acid A, i.e. the mixture of enantiomers and the carboxylic acid A are dissolved in a suitable solvent or solvent mixture prior to crystallization. The solution can be a homogenous solution, i.e. the mixture of enantiomers, the carboxylic acid A and at least one solvent form a single phase prior to crystallization, or an heterogenous solution or emulsion, wherein the mixture of enantiomers, the carboxylic acid A are dissolved in at least one solvent, preferably in a mixture of at least two solvents of different polarity, thereby forming a multi-phase liquid. Thus, the crystallization can be performed either from a homogeneous solution containing the mixture of the enantiomers of formula I and the achiral carboxylic acid A in a suitable solvent, or from an emulsion in which the mixture of the enantiomers of formula I and the achiral carboxylic acid A is dissolved. Both, the homogenous solution and the emulsion may further comprise solid material, in particular undissolved carboxylic acid A and/or compound of the formula I or inert solid material, which is insoluble or sparingly soluble in the solvent of choice.

For crystallization from a homogeneous solution, the mixture of the enantiomers of the compound of formula I, either in the form of its free base or in the form of its acid-addition salt, is dissolved in a suitable solvent. If the free base is used, the necessary amount of the achiral carboxylic acid A is added to the solution.

Suitable solvents include in particular water, organic solvents that have a miscibility with water of at least 20 % at room temperature, and mixtures thereof with water. Suitable organic solvents that have a miscibility with water of at least 20 % at room temperature include:
1. C₁-C₄-alkanols such as methanol, ethanol, n-propanol, isopropanol,;
2. amides and N,N-dimethylamides of C₁-C₃-carboxylic acids such as formamide, dimethylformamide (DMF), acetamide and N,N-dimethylacetamide;
3. 5 or 6-membered lactames with a total of 7 carbon atoms such as pyrrolidone, N-methylpyrrolidone, N-ethylpyrrolidone, N-isopropylpyrrolidone, N-hydroxyethylpyrrolidone;
4. dimethylsulfoxid and sulfolane;
5. acetonitril;
6. 5- or 6-membered lactones such as γ-butyrolactone;
7. polyols and polyetherols such as glycol, glycerin, dimethoxyethan, ethylendiglycol, ethylenglycolmonomethylether, etc.

In a preferred embodiment of the invention, the crystallization of the acid-addition salt of the compound of formula I is performed from an aqueous solution, i.e. the compound of formula I and the carboxylic acid A are dissolved in an aqueous solvent. The term "aqueous solvent" includes water and mixtures of water with organic solvents having a water solubility of at least 20 % v/v at room temperature, wherein water is the main constituent, The term "aqueous solvent" in particular relates in particular to water and mixtures thereof with said organic solvents, wherein the amount of said organic solvent does not exceed 20 % v/v, in particular 10 % v/v, based on the total amount of solvent used.

In another preferred embodiment of the invention, the crystallization of the acid-addition salt of the compound of formula I is performed from a homogeneous solution containing the mixture of enantiomers and the carboxylic acid A in a C₁-C₄-alkanol, like methanol or ethanol, or a mixture thereof or a mixture thereof with water containing at least 50 % v/v, in particular at least 80 % v/v of at least one C₁-C₄-alkanol.

For crystallization from a homogeneous solution, the solution of the mixture of the enantiomers of the formula I and the necessary amount of achiral carboxylic acid A is supersaturated, and then crystallization is induced as described above.

Supersaturation can be achieved by standard methods, for example by dissolving the mixture of enantiomers and, if necessary, the achiral carboxylic acid A in the respective solvent or solvent mixture with heating in an amount so that the obtained solution is saturated or nearly saturated. Then, the obtained solution is cooled down below the saturation temperature, whereby a supersaturated solution is obtained. Likewise, supersaturation can be achieved by evaporating solvent from a non-saturated solution until the concentration of the acid-addition salt of the compound of formula I is higher than the concentration of a saturated solution. It is likewise possible to obtain a supersaturated solution by adding more achiral carboxylic acid A in order to reduce the solubility of the acid-addition salt of the enantiomers of formula I in the solution. Of course, any of these measures can be combined.

In the thus obtained supersaturated solution, crystallization is induced as described above. In particular, crystallization is induced by adding seed crystals of the acid-addition salt of the desired enantiomer. The seed crystals can be added as such or in the form of a suspension in the solvent. The particle size of the seed crystals may range from 0.5 to 20 µm, preferably from 0.5 to 15 µm, in particular from 0.5 to 10 µm. It can be advantageous to further lower the temperature of the solution after the seed crystals have been added. During crystallization, the temperature can be reduced and/or further solvent can be evaporated in order to assist the crystallization. In a preferred embodiment, the temperature during crystallization is lowered by at least 5 K, preferably at least 10 K, e.g. by 10 to 20 K. Usually, the crystallization process is performed at a temperature ranging from -20 to +80° C, in particular from 0 to 50° C. The crystallization process can be further optimized by a proper agitation during the crystallization, e. g. by stirring, shaking or pumping.

The concentration of the enantiomers of formula I prior to crystallization from the homogeneous solution may vary, depending on the solvent and the carboxylic acid A. Usually, the concentration ranges from 25 to 700 g/L, preferably from 50 to 650 g/L, in particular from 100 to 500 g/L, more preferably from 150 to 450 g/L, calculated as the free base of I.

In another preferred embodiment, the crystallization is effected by so-called "emulsion crystallization". Emulsion crystallization is a well-established technique and known e. g. from EP 548028, WO 97/32644, WO 99/12623, WO 00/54865 and WO 00/53283.

In emulsion crystallization, the crystallization of the desired enantiomer of the acid-addition salt is effected from a supersaturated emulsion containing the mixture of the enantiomers in the form of their acid-addition salts with the carboxylic acid A. The emulsion comprises a continuos phase formed by at least one first solvent and a discontinuous phase (droplets) formed by at least one second solvent. The acid addition salt of the compound of formula I may be present in both phases. The discontinuous phase may be formed by a non-polar (lipophilic) solvent or an amphiphilic solvent or a mixture thereof, while the continuos phase is formed by a highly polar organic solvent or water or a mixture thereof. However, emulsions where the discontinous phase is formed by the polar solvent and the continous phase is formed by the non-polar and/or amphiphilic solvent, are likewise suitable for the purpose of the invention

The emulsion can be a macroemulsion, i. e. the average droplet size is > 500 nm, in particular > 1 µm, or a microemulsion, i. e. the average droplet size is < 500 nm, in particular < 300 nm. Crystallization from microemulsions is preferred. The droplet size can be determined by light scattering methods.

In the emulsions, the relative phase volume of the disperse phase may vary and preferably lies in a range from 5 to 65 % v/v, in particular from 10 to 50 % v/v, based on the total volume of the emulsion.

The emulsion will usually contain one or more surfactant as described above, which assists in forming and stabilizing the emulsion droplets. The surfactants can be non-ionic, anionic, cationic or zwitterionic. Preferably, the surfactant, if present, comprises at least 50 % by weight, based on the total amount of surfactant present, of at least one non-ionic surfactant. In particular, the surfactant comprises at least 80 %, based on the total amount of surfactant, of at least one non-ionic surfactant. The surfactant will normally be present in an amount ranging from 0.1 to 30 % by weight, based on the total weight of the emulsion, in particular from 1 to 20 % by weight.

For obtaining an emulsion containing the enantiomers of the acid-addition salt, said mixture of enantiomers can be dissolved in a suitable mixture of solvents, in particular a mixture of water and organic solvent. The mixture usually contains a surface active agent suitable for stabilizing the droplets of the emulsion. It is likewise possible to dissolve the enantiomeric mixture of the acid-addition salt or the free base and the achiral carboxylic acid A in a suitable solvent and then emulsify the obtained solution together with a surfactant in water or another solvent, which is suitable for forming an emulsion.

The non polar solvents are organic solvents having a water solubility of ≤ 5 % v/v at room temperature. Amphiphilic solvents are organic solvents that are soluble in both non-polar, lipophilic and polar, hydrophilic phases. They have a water solubility of > 5 % v/v at room temperature and a solubility of > 5 % v/v at room temperature in methyloleate. Highly polar solvents include water and organic solvents which have a solubility of ≤ 5 % v/v at room temperature in methyloleate.

Suitable solvents for emulsion crystallization include
I. non-polar, lipophilic solvents, such as
   1. aromatic solvents, such as benzene or its derivatives like toluene, benzonitrile, nitrobenzene, chlorobenzene or xylene and heteroaromatic liquids such as pyridine or furane;
   2. halogenated alkanes like dichloromethane, dichloroethane, trichloroethane;
   3. alkyl ethers, with ≥ 4 carbon atoms, such as diethylether or tert-butylmethylether;
   4. esters such as n-, i- or branched with ≥ 5 carbon atoms, including diesters, triesters, such as oils and fats, and polyesters;
   5. alkanoles, aromatic and cyclic alcohols with ≥ 5 carbon atoms, e.g. 2-hexanol, cyclohexanol, benzylalcohol or octanol;
   6. alkanes such as n-, i- or branched, including cycloalkanes (e.g. cyclohexane, methylcyclohexane).
II. amphiphilic solvents, such as
   1. ethers such as tetrahydrofurane, dimethoxyethane, dioxane, trioxane, diethyleneglycoledimethylether, triethyleneglycoledimethylether, dipropyleneglycoldimethylether, low molecular weight polyethyleneglycoles and low molecular weight polypropyleneglycoles (MW ≤ 400);
   2. alkanols and cycloalkanols with 1 to 5 carbon atoms such as methanol, ethanol, isopropanol, isobutanol, cyclobutanol and cyclopentanol;
   3. aminoalcohols such as ethanolamine, diethanolamine and triethanolamine;
   4. ketones with 3 to 6 carbon atoms such as acetone, 2-butanone, cyclopentanone and cyclohexanone;
   5. esters of C₁-C₃-alkanoic acids with C₁-C₄-alkanols having a total ≤ 4 carbon atoms such as methyl and ethyl acetate, methyl propionate, methyl-, ethyl- and propyl formiate, the mono- and diacetates of ethylenglycol, propylenglycol, diethylenglycol or triethylenglycol;
   6. lactones such as γ-butyrolactone;
   7. amides and N,N-dimethylamides of C₁-C₃-carboxylic acids such as formamide, dimethylformamide (DMF), acetamide and N,N-dimethylacetamide;
   8. 5 or 6-membered lactames such as pyrrolidone, N-methylpyrrolidone, N-ethylpyrrolidone, N-isopropylpyrrolidone, N-hydroxyethylpyrrolidone, n-propylpyrrolidone, n-octylpyrrolidone.
III. highly polar solvents, such as water or dimethylsulfoxid (DMSO).

In a preferred embodiment of the emulsion crystallization, the crystallization is performed from an aqueous emulsion, i.e. the emulsion comprises water and at least one organic solvent. The amount of organic solvent may vary and is preferably from 5 to 65 % w/w, in particular from 20 to 55 % w/w, based on the total amount of organic solvent and water. Preferred organic solvents for emulsion crystallization from an aqueous emulsion are selected from the group of amphiphilic solvents II, in particular from the groups 11.2, 11.4, 11.6, 11.7 and 11.8.

The emulsion may further contain additives such as anti-foaming agents, solubility regulating additives, anti-freeze agents and the like. Agents for adjusting the solubility and/or the freezing point of the aqueous phase include e.g. a water-miscible organic liquid such as a C₁-C₃-alkanol, dimethyl sulfoxide, acetonitrile, etc.

For crystallization from the emulsion, the emulsion containing the mixture of the enantiomers of formula I and the necessary amount of achiral carboxylic acid A is supersaturated, and then crystallization is induced as described above.

Supersaturation can be achieved in a manner similar to that of the methods described above, preferably by dissolving the mixture of enantiomers and, if necessary, the achiral carboxylic acid A in the respective solvent or solvent mixture with heating in an amount so that the obtained emulsion is saturated or nearly saturated and cooling the obtained emulsion below the saturation temperature, whereby a supersaturated emulsion is obtained. It is likewise possible to obtain a supersaturated emulsion by adding further achiral carboxylic acid A in order to reduce the solubility of the acid-addition salt of the enantiomers of formula I in the emulsion or by the use of ultrasound. Of course, any of these measures can be combined.

In the thus obtained supersaturated emulsion, crystallization is induced as described above. In particular, crystallization is induced by adding seed crystals of the acid-addition salt of the desired enantiomer to the emulsion. The seed crystals can be added as such or in the form of a suspension in a solvent or in emulsion. The particle size of the seed crystals may range from 0.5 to 20 µm, preferably from 0.5 to 15 µm, in particular from 0.5 to 10 µm. It can be advantageous to further lower the temperature of the emulsion after the seed crystals have been added. During crystallization, the temperature can be reduced and/or further solvent can be evaporated in order to assist the crystallization. In a preferred embodiment, the temperature during emulsion crystallization is kept constant or nearly constant (± 2K). Usually, the crystallization process is performed at a temperature ranging from -20 to +80° C, in particular from 0 to 50° C. The crystallization process can be further optimized by proper agitation during the crystallization, e. g. by stirring, shaking or pumping.

The concentration of the mixture of enantiomers of formula I in the emulsion before crystallization starts may range from 25 to 700 g/L, preferably from 50 to 650 g/L, in particular from 100 to 500 g/L, more preferably from 150 to 450 g/L, calculated as the free base of I.

Irrespective of the crystallization technique used, the crystallization of the desired enantiomer of the acid-addition salt is usually performed such that at least 5 g/L of the desired enantiomer of the acid-addition salt of a compound of formula I has been crystallized. Preferably, depending on the salbutamol derivative, the carboxylic acid A, the concentration and the level of supersaturation used, the crystallization is performed in such a way, that 5 to 200 g/L, in particular 10 to 100 g/L of the desired enantiomer of the acid-addition salt, is obtained.

The crystalline material is separated from the mother liquor by usual solid/liquid separation techniques, including filtration and centrifugation. After the removal of the mother liquor, the obtained solid material is usually washed with water and/or a suitable organic solvent to remove residual mother liquor.

The thus obtained crystalline product usually has an enantiomeric ratio of at least 70 : 30, in particular at least 75 : 25, and more preferably at least 80 : 20. In order to further enrich the desired enantiomer, the obtained crystalline material can be subjected to a further conventional re-crystallization. The re-crystallization of the acid-addition salt can be performed from a homogeneous solution of the acid-addition salt or from an emulsion as described above. In particular, the acid-addition salt obtained from the preferential crystallization is re-crystallized from a solvent, selected from water, organic solvents having a water miscibility of at least 20 % v/v at room temperature and mixtures thereof with water. More preferably, the solvent used for re-crystallization is selected from water, C₁-C₄-alkanols such as methanol or ethanol or mixtures thereof with water.

Both the preferential crystallization and the re-crystallization may be carried out as a batch process or as a continuous process. One means for carrying out the process continuously is illustrated in figure 1 of WO 97/32644, to which full reference is made.

The mother liquor obtained in the first crystallization according to the present invention is depleted with regard to the desired enantiomer and thus contains an excess of the non-desired enantiomer. In a preferred embodiment of the invention, said mother liquor is subjected to a further crystallization in order to crystallize the acid-addition salt of the non-desired enantiomer, in particular the acid-addition salt of the S-enantiomer of a salbutamol or 4-benzyl salbutamol.

In order to achieve the crystallization of the non-desired enantiomer, the mother liquor has to be supersaturated. Supersaturation of the mother liquor may be achieved by further reducing the temperature of the mother liquor, by concentrating the mother liquor, or by further adding the mixture of the enantiomers of formula I and the achiral carboxylic acid A (either separately or as the acid-addition salt). The crystallization of the non-desired enantiomer is then carried out as outlined above. Thereby, the mother liquor is obtained which contains an excess of the desired enantiomer. This mother liquor can be subjected to a further crystallization as outlined above, usually after addition of the required amount of the mixture of enantiomers to be separated to reach the original concentration and the corresponding amount of achiral carboxylic acid A. These steps of consecutive crystallization of the desired enantiomer and non-desired enantiomer can be repeated until the yield is nearly quantitative . The process of consecutive crystallization allows a very efficient separation of the enantiomers of salbutamol, 4-benzyl salbutamol and its derivatives.

The desired enantiomer of the acid-addition salt of salbutamol or 4-benzyl salbutamol can be transformed into a pharmaceutically acceptable acid-addition salt by standard techniques as described in the prior art cited in the introductory part of the present application. For preparation of a pharmaceutically acceptable acid-addition salt, such as the sulfate or hydrochloride, the acid-addition salt obtained in the crystallization according to the invention is transformed into the free base and then treated with an acid that provides the pharmaceutical acceptable counter ion. The transformation of the acid-addition salt of the compound of formula I into its free bases is usually achieved by treatment with a diluted aqueous base, in particular an aqueous solution of an alkalimetal carbonate, alkalimetal hydrogen carbonate or alkalimetal hydroxide such as sodium carbonate, potassium carbonate, sodium hydrogencarbonate, sodium hydroxide, calcium hydroxide or potassium hydroxide, optionally in the presence of an organic solvent to assist solubilization of the free base. Optionally, the carboxylic acid is removed with a basic ion exchange resin. The thus obtained free base is then treated with a solution of the desired acid in water or in a suitable organic solvent. It is, however, also possible to transform the acid-addition salt obtained from the crystallization according to the present invention into the pharmaceutically acceptable acid-addition salt by direct treatment of the acid-addition salt with an acid that provides the pharmaceutically acceptable counter ion, e. g. the acid-addition salt obtained from the crystallization is dissolved in a suitable solvent, e. g. in a C₁-C₄-alkanol and the acid providing the pharmaceutical acceptable counter ion is added to precipitate the pharmaceutically acceptable acid-addition salt.

If 4-benzyl salbutamol is separated into its enantiomers according to the process of the present invention, the obtained acid-addition salt of 4-benzyl salbutamol or the free base can be subjected to a conventional debenzylation with no occurrence of racemisation. Suitable methods are known in the art, e. g. from EP 1349828. Usually the acid-addition salt of the desired enantiomer of 4-benzyl salbutamol, if it is stable under the cleavage conditions, or its free base is suspended or dissolved in a suitable organic solvent such as ethanol and hydrogenated in the presence of a suitable transition metal catalyst such as palladium on charcoal or Raney nickel. The thus obtained free base or acid-addition salt of salbutamol can be transferred into a pharmaceutically acceptable acid-addition salt as described above.

The non-desired S-enantiomer of salbutamol or 4-benzyl salbutamol can be racemized by standard techniques, e. g. by treatment with a strong acid such a sulfuric acid or toluene sulfonic acid at elevated temperature. In particular, racemisation can be performed by analogy to example 15 described in US 6,365,756. The thus obtained racemized mixture of salbutamol or 4-benzyl salbutamol enantiomers can be subjected to a crystallization according to the present invention, thereby improving the overall yield of the desired enantiomer.

The following examples shall serve the further illustration of the invention and are not intended to limit the scope of the present invention.

### Example 1: Crystallization of R-salbutamol*pivalic acid from a homogenous solution of the racemate

Racemic salbutamol (1.88g; 7.8 mmol) and pivalic acid (0.80g; 7.8 mmol) were dissolved in water (5.9g) at 60°C. The solution was cooled to 38°C and seeded with R-salbutamol*pivalic acid crystals (8 mg; 0.023 mmol). The mixture was cooled to room temperature under stirring. After stirring for 16 h at room temperature the crystals were filtered to yield R-salbutamol*pivalic acid (354mg; 12.9%) with an enantiomeric ratio of 84.4:15.6 er. (HPLC).

### Example 2: Crystallization of R-salbutamol*pivalic acid from an emulsion of the racemate

Racemic salbutamol (1.55g; 6.5 mmol) and pivalic acid (0.66g; 6.5 mmol) were dissolved in an emulsion (3.63g) of water/N-methylpyrrolidone/surfactant 1/isobutanol (40:40:10:10 w/w) at 60°C. The solution was cooled to 22°C and seeded with R-salbutamol*pivalic acid crystals (8 mg; 0.023 mmol). The mixture was cooled to 20°C under stirring. After stirring for 4 h at 20°C the crystals were filtered, washed with isobutanol (0.5 ml) and dried to yield R-salbutamol*pivalic acid (297mg; 13.1 %) with an enantiomeric ratio of 89.8:10.2 er. (HPLC).

Surfactant 1 was a polyethoxylate of nonylphenol (HLB >10): Synperonic NP10

### Example 3: Crystallization of S-salbutamol*pivalic acid from mother liquor

To the mother liquor of example 2 (3.1g, salbutamol content: 5.3mmol) racemic salbutamol (175mg; 0.73mmol) and pivalic acid (75mg; 0.73mmol) were added and the mixture was heated to 60°C. The obtaind clear solution was cooled to 22°C and seeded with S-salbutamol*pivalic acid crystals (8 mg; 0.023 mmol). The mixture was cooled to 20°C under stirring. After stirring for 3 h at 20°C the crystals were filtered, washed with isobutanol (0.5 ml) and dried to yield S-salbutamol*pivalic acid (260 mg; 13%) with an enantiomeric ratio of 88:12 er. (HPLC).

### Example 4: Crystallization of R-salbutamol*pivalic acid from an emulsion of the racemate

Racemic salbutamol (1.16g; 4.9 mmol) and pivalic acid (0.50g; 4.9 mmol) were dissolved in an emulsion (2.36g) of water/N-methylpyrrolidone/surfactant 2/isobutanol (40:40:10:10 w/w) at 60°C. The solution was cooled to 21°C and seeded with R-salbutamol*pivalic acid crystals (5 mg; 0.015 mmol). After stirring for 90 min at 21 °C the crystals were filtered, washed with isobutanol (0.4 ml) and dried to yield R-salbutamol*pivalic acid (110mg; 6.3%) with an enantiomeric ratio of 75:25 er.

Surfactant 2 was a polyethoxylate of sorbitan fatty acid ester (HLB 15): Tween 80

### Example 5: Recrystallization of R-salbutamol*pivalic acid

R-Salbutamol*pivalic acid (1.73g, 5.1 mmol) with an enantiomeric ratio of 75:25 er was dissolved at 60°C in water (2.1g). After cooling to room temperature the mixture was stirred for 6h. The obtained crystals were filtered, washed with water and dried to yield R-salbutamol*pivalic acid (0.86g, 2.5 mmol) with an enantiomeric ratio of 97:3 er. (HPLC).

Repeated recrystallistion of this R-salbutamol*pivalic acid material (enantiomeric ratio 97:3 er) provided R-salbutamol*pivalic acid with an enantiomeric excess of > 99% ee.

### Example 6: Transformation of R-salbutamol*pivalic acid into (salbutamol)₂*H₂SO₄

A mixture of methanol (0.31 g) and sulfuric acid (96%; 32mg; 0.31 mmol) was added to a solution of R-salbutamol*pivalic acid (213mg; 0.62 mmol) in methanol (0.31g) and the obtained mixture was strirred for 30min. The crystals were filtered and washed with methanol to yield (salbutamol)₂*H₂SO₄ (162mg; 0.28mmol; 90% of th.).

### Example 7: Crystallization of S-4-benzyl-salbutamol*3,5-dinitro-benzoic acid from an emulsion of the racemate

Racemic 4-benzyl-salbutamol (0.86g; 2.62 mmol) and 3,5-dinitro-benzoic acid (0.56g; 2.62 mmol) were dissolved in an emulsion (2.5g) of water/N-methylpyrrolidone/surfactant 1/isobutanol (40:40:10:10 w/w) at 60°C. The solution was cooled to 24°C and seeded with S-4-benzyl-salbutamol*3,5-dinitro-benzoic acid crystals (12 mg; 0.022 mmol). The mixture was cooled to 22°C under stirring over a period of 20 min. Then, the crystals were filtered, washed with isobutanol (0.5 ml) and dried to yield S-4-benzyl-salbutamol*3,5-dinitro-benzoic acid (160mg; 10.4%) with an enantiomeric ratio of 92.4:7.6 er. (HPLC).

Surfactant 1 was a polyethoxylate of nonylphenol (HLB >10): Synperonic NP10

### Example 8: Crystallization of S-4-benzyl-salbutamol*3,5-dinitro-benzoic acid from a homogenous solution of the racemate

Racemic 4-benzyl-salbutamol (0.86g; 2.62 mmol) and 3,5-dinitro-benzoic acid (0.56g; 2.62 mmol) were dissolved in methanol (1.5g) at 60°C. The solution was cooled to 35°C and seeded with S-4-benzyl-salbutamol*3,5-dinitro-benzoic acid crystals (12 mg; 0.022 mmol). The mixture was cooled to 21 °C under stirring over a period of 30 min and stirred at 21 °C for 18 h. Then, the crystals were filtered, washed with methanol (0.2 ml) and dried to yield S-4-benzyl-salbutamol*3,5-dinitro-benzoic acid (213mg; 14.2%) with an enantiomeric ratio of 90.9:9.1 er. (HPLC).

### Example 9: Recrystallization of S-4-benzyl-salbutamol*3,5-dinitro-benzoic acid

S-4-Benzyl-salbutamol*3,5-dinitro-benzoic acid (178mg, 0.33 mmol) with an enantiomeric ratio of 90.9:9.1 er was dissolved at 60°C in a mixture of water and methanol (2:1 w/w%; 2.2g). After cooling to room temperature the mixture was stirred for 30 min. The obtained crystals were filtered, washed with water and dried to yield S-4-Benzyl-salbutamol*3,5-dinitro-benzoic acid (125mg, 0.23 mmol) with an enantiomeric ratio of 98:2 er. (HPLC).

Repeated recrystallistion of this S-4-Benzyl-salbutamol*3,5-dinitro-benzoic acid (enantiomeric ratio 98:2 er) provided S-4-Benzyl-salbutamol*3,5-dinitro-benzoic acid with an enantiomeric ratio of > 99:1 er.

## Claims

1. A process for enantiomeric enrichment of a compound of the formula I or an acid-addition salt thereof 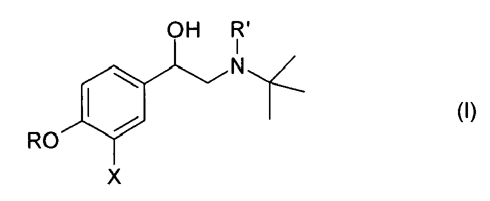 where R is hydrogen or benzyl, R' is hydrogen or benzyl and X is CH₂OH or COO-C₁-C₄-alkyl
the process comprising the crystallization of a compound of the formula I in the form of its acid-addition salt with an achiral carboxylic acid A that has at least three carbon atoms and a solubility of less than 50 g/l in water at pH < 3, 20°C and 1013 mbar, from a solution containing a mixture of the enantiomers of the compound of formula I and the achiral carboxylic acid A in the presence of seed crystals of the desired enantiomer of the compound of formula I or the acid addition salt thereof, whereby the enantiomerically enriched acid-addition salt of the compound of formula I is obtained.

2. The process according to claim 1, wherein the carboxylic acid A is selected from alkanoic acids having from 4 to 20 carbon atoms, benzoic acids and naphthoic acids, where the benzene or naphthene ring may carry 1, 2 or 3 radicals selected, independently of each other, from halogen, alkyl, cyano, nitro, hydroxy and alkoxy.

3. The process according to claim 2, wherein the carboxylic acid A is an α-branched alkanoic acid, in particular pivalic acid.

4. The process according to claim 2, wherein the carboxylic acid A is a benzoic acid, in particular 3,5-dinitrobenzoic acid.

5. The process according to any of the preceding claims, wherein R' in formula I is hydrogen and X is CH₂OH.

6. The process according to any of the preceding claims, wherein the compound I is present in the solution before crystallization as its racemic mixture.

7. The process according to any of the preceding claims, wherein the crystallization is effected from a supersaturated solution of the acid-addition salt in a solvent, selected from water, water-miscible organic solvents and mixtures thereof.

8. The process according to any of claims 1 to 6, wherein the crystallization is effected from a supersaturated emulsion.

9. The process according to any of the preceding claims, comprising
i) Crystallizing the compound of the formula I in the form of its acid-addition salt with the achiral carboxylic acid A, whereby the acid-addition salt is obtained in the form of a crystalline material that is enriched with the desired enantiomer, and removing the obtained crystalline material from the mother liquor;
ii) Supersaturating the mother liquor and inducing crystallization of the non-desired enantiomer from the mother liquor.

10. The process according to any of the preceding claims, further comprising a recrystallization of the enantiomerically enriched acid-addition salt obtained.

11. The process according to any of the preceding claims, further comprising the transformation of the enantiomerically enriched acid-addition salt obtained into the sulfate, hydrogen sulfate or hydrochloride of the compound of formula 1.

12. An enantiomerically enriched or pure acid-addition salt of a compound of the formula I as defined in claim 1 with a carboxylic acid having at least three carbon atoms and a solubility of less than 50 g/I in water at pH < 3, 20°C and 1013 mbar.

13. The acid-addition salt according to claim 12 with pivalic acid.

14. The acid-addition salt according to claim 12 with 3,5-dinitrobenzoic acid.
